# EUROPEAN PATENT SPECIFICATION

(11) **EP 1 206 284 B1**
(45) Date of publication and mention of the grant of the patent: **11.07.2007**
(21) Application number: 00951707.9
(22) Date of filing: 09.08.2000
(51) Int. Cl.: A61K 47/36, A61K 48/00, C12N 15/86, C12N 15/11

(54) **METHOD FOR DELIVERY OF THERAPEUTIC AGENTS USING A SOLUTION OF DEXTRIN**
VERFAHREN FÜR DIE VERABREICHUNG VON HEILMITTELN MITTELS EINER DEXTRINLÖSUNG
METHODE D'ADMINISTRATION D'AGENTS THERAPEUTIQUES UTILISANT UNE SOLUTION DE DEXTRINE

(30) Priority: 10.08.1999 GB 9918785; 22.10.1999 GB 9924980; 13.01.2000 US 482794
(43) Date of publication of application: 22.05.2002
(73) Proprietor: Innovata PLC, Ruddington Nottinghamshire NG11 6JS (GB)
(72) Inventor: CONROY, Susan, London W1R 9AJ (GB); BOYES, Robert Nichol, London W1R 9AJ (GB)
(74) Representative: Harrison Goddard Foote
(86) International application number: PCT/GB2000/003025
(87) International publication number: WO 2001/012231

(56) References cited:
- WO-A-95/34325
- WO-A-97/10005
- GB-A- 2 207 050
- US-A- 5 686 289
- CROYLE MARIA A ET AL: "Beta cyclodextrins enhance adenoviral- mediated gene delivery to the intestine" PHARMACEUTICAL RESEARCH,NEW YORK, NY,US, vol. 15, no. 9, 1998, pages 1348-1355, XP000953222 ISSN: 0724-8741
- E. PEERS AND R. GOKAL: "Icodextrin provides long dwell peritonela dialysis and maintenance of intraperitoneal volume" ARTIFICIAL ORGANS, vol. 22, no. 1, January 1998 (1998-01), pages 8-12, XP000945076
- MARTIS L ET AL: "PERITONEAL DIALYSIS SOLUTIONS FOR THE 21ST CENTURY" ARTIFICIAL ORGANS,US,BLACKWELL SCIENTIFIC PUBLICATIONS, INC., BOSTON, vol. 22, no. 1, 1998, pages 13-16, XP000901321 ISSN: 0160-564X
- DATABASE BIOSIS [Online] BIOSCIENCES INFORMATION SERVICE, PHILADELPHIA, PA, US; March 2000 (2000-03) CONROY SUSAN E ET AL: "In vitro viral vector stability and fluid dynamics of an intraperitoneal solution for delivery of gene therapy." Database accession no. PREV200000227612 XP002155779 & PROCEEDINGS OF THE AMERICAN ASSOCIATION FOR CANCER RESEARCH ANNUAL, no. 41, March 2000 (2000-03), page 524 91st Annual Meeting of the American Association for Cancer Research.;San Francisco, California, USA; April 01-05, 2000, March, 2000 ISSN: 0197-016X

## Description

### FIELD OF THE INVENTION

This invention relates to therapeutic treatment and in particular to the delivery of viral based vectors to an animal subject, including a human being, via a body cavity of that subject. The viral based vectors may be active in a variety of ways, for instance, in connection with gene therapy and immuno therapy.

### BACKGROUND OF THE INVENTION

Biologically active agents may be introduced into an animal subject in a variety of ways including enterally (orally, rectally or sublingually) or parenterally (intravenously, subcutaneously, or by inhalation).

This invention is concerned with the parenteral administration of viral based vectors and in particular by the introduction of a viral based vector to the animal subject via a body cavity such as the peritoneum or the ocular cavity. Reference will be made hereinafter to the peritoneum, but it should be understood that the invention has application to the delivery of viral based vectors via other body cavities.

It is known that introduction of certain aqueous solutions into the peritoneal cavity can be useful in the treatment of patients suffering from renal failure. Such treatment is known as peritoneal dialysis. The solutions contain electrolytes similar to those present in plasma; they also contain an osmotic agent, normally dextrose, which is present in a concentration sufficient to create a desired degree of osmotic pressure across the peritoneal membrane. Under the influence of this osmotic pressure, an exchange takes place across the peritoneal membrane and results in withdrawal from the bloodstream of waste products, such as urea and creatinine, which have accumulated in the blood due to the lack of normal kidney function. While this exchange is taking place, there is also a net transfer of dextrose from the solution to the blood across the peritoneal membrane, which causes the osmolality of the solution to fall. Because of this, the initial osmolality of the solution must be made fairly high (by using a sufficiently high concentration of dextrose) in order that the solution continues to effect dialysis for a reasonable length of time before it has to be withdrawn and replaced by fresh solution.

Other osmotic agents have been proposed for use in peritoneal dialysis and in recent years dextrin (a starch hydrolysate polymer of glucose) has been used. When instilled in the peritoneal cavity, dextrin is slowly absorbed via the lymphatic system, eventually reaching the peripheral circulation. The structure of dextrin is such that amylases break the molecule down into oligosaccharides in the circulation. These are cleared by further metabolism into glucose.

Dextrin solutions have been proposed as the medium for delivery of drugs to the body via the peritoneum. In GB-A-2207050, such a solution is proposed for the intraperitoneal administration of drugs for which enteral administration is unsatisfactory. Such an approach is stated to be particularly useful for the delivery of peptide drugs such as erythropoetin and growth hormones. Reference is also made to cephalosporin antibiotics. The concentration of dextrin in the aqueous solution is stated to be preferably from 0.5 to 10% w/v and an example of a composition for the delivery of erythropoetin has a dextrin concentration of about 10% w/v.

Gene therapy is concerned, inter alia, with the transfer of genetic material to specific target cells of a patient to prevent or alter a particular disease state. The treatment involves the use of carriers or delivery vehicles, often termed vectors, adapted for the delivery of therapeutic genetic material. These vectors are usually viral but non-viral vectors are also known. Immunogene therapy involves the use of genes for immunotherapy, including the provision of gene-based vaccines.

The mesothelial lining of the peritoneal cavity comprises a lining of cells that cover a broad surface. The peritoneal mesothelium has good lymphatic drainage and permits diffusion of macromolecules. Adenovirus-mediated gene transfer to the peritoneal mesothelium in the rat has been shown to be feasible (Setoguchi et al. Intraperitoneal *in vivo* Gene Therapy to Deliver α1-antitrypsin to the systemic circulation. (American Journal of Respiratory Cellular Molecular Biology, 994;10: 369-377).

Typically, a medium chosen to introduce gene therapy materials to a patient via a body cavity might be a buffered saline solution, for instance, a viral phosphate buffered saline (vPBS). However, the use of such a solution has not proved to be particularly effective, problems arising in connection with the stability of the solution, the dwell time in the body cavity as well as the effectiveness of transgene expression.

### STATEMENTS OF INVENTION

The present invention provides composition for delivering a viral based vector to an animal subject, the method comprising introducing into a body cavity of the animal subject the viral based vector and a dextrin solution.

The present invention is therefore not concerned with biologically active agents which are in the nature of drugs such as those with which GB-A-2207050 is concerned. Rather, it is concerned with viral based vectors which act indirectly such as gene therapy agents and immunotherapy agents. The latter include, for instance, immunotherapeutic agents relating to cytokine genes. Agents with which the invention is concerned include genes carried by or encapsulated within viral vectors and liposomes/cationic lipids as well as constructs such as a conjugate of Interleukin-2 and a biologically active agent such as a gene. Vectors are typically adapted for expression of gene carried by the vector.

Typically said adaptation includes, by example and not by way of limitation, the provision of transcription control sequences (promoter sequences) which mediate cell/tissue specific expression. These promoter sequences may be cell/tissue specific, inducible or constitutive.

Promoter is an art recognised term and, for the sake of clarity, includes the following features which are provided by example only, and not by way of limitation. Enhancer elements are cis acting nucleic acid sequences often found 5' to the transcription initiation site of a gene ( enhancers can also be found 3' to a gene sequence or even located in intronic sequences). Enhancers function to increase the rate of transcription of the gene to which the enhancer is linked. Enhancer activity is responsive to trans acting transcription factors (polypeptides) which have been shown to bind specifically to enhancer elements. The binding/activity of transcription factors (please see Eukaryotic Transcription Factors, by David S Latchman, Academic Press Ltd, San Diego) is responsive to a number of physiological/environmental cues which include, by example and not by way of limitation, intermediary metabolites (eg glucose, lipids), environmental effectors ( eg light, heat,).

Promoter elements also include so called TATA box and RNA polymerase initiation selection (RIS) sequences which function to select a site of transcription initiation. These sequences also bind polypeptides which function, *inter alia,* to facilitate transcription initiation selection by RNA polymerase.

Adaptations also include the provision of selectable markers and autonomous replication sequences which facilitate the maintenance of said vector in either the eukaryotic cell or prokaryotic host. Vectors which are maintained autonomously are referred to as episomal vectors. Episomal vectors are desirable since these molecules can incorporate large DNA fragments (30-50kb DNA). Episomal vectors of this type are described in WO98/07876.

Adaptations which facilitate the expression of vector encoded genes include the provision of transcription termination/polyadenylation sequences. This also includes the provision of internal ribosome entry sites (IRES) which function to maximise expression of vector encoded genes arranged in bicistronic or multi-cistronic expression cassettes. Expression control sequences also include so-called Locus Control Regions (LCRs). These are regulatory elements which confer position-independent, copy number-dependent expression to linked genes when assayed as transgenic constructs in mice. LCRs include regulatory elements that insulate transgenes from the silencing effects of adjacent heterochromatin, Grosveld et al., Cell (1987), 51:975-985.

Expression control sequences also encompass, ubiquitous chromatin opening elements (LTCOE's), see WO/GB00/05393. UCOE's are nucleic acid elements that are responsible for establishing an open chromatin structure across a locus that consists exclusively of ubiquitously expressed, housekeeping genes. These elements are not derived from an LCR. A UCOE is a polynucleotide which opens chromatin or maintains chromatin in an open state and facilitates reproducible expression of an operably-linked gene in cells of at least two different tissue types.

These adaptations are well known in the art. There is a significant amount of published literature with respect to expression vector construction and recombinant DNA techniques in general. Please see, Sambrook et al (1989) Molecular Cloning: A Laboratory Manual, Cold Spring Harbour Laboratory, Cold Spring Harbour, NY and references therein; Marston, F (1987) DNA Cloning Techniques: A Practical Approach Vol III IRL Press, Oxford UK; DNA Cloning: F M Ausubel et al, Current Protocols in Molecular Biology, John Wiley & Sons, Inc. (1994).

Vectors are viral based and include by example and not by way of limitation the following: adenovirus; retrovirus; adeno-associated virus; herpesvirus; lentivirus; vaccinia virus; baculovirus.

The invention also encompasses viral based vectors including antisense nucleotide sequences, including antisense oligonucleotides.

As used herein, the term "antisense oligonucleotide" or "antisense" describes an oligonucleotide that is an oligoribonucleotide, oligodeoxyribonucleotide, modified oligoribonucleotide, or modified oligodeoxyribonucleotide which hybridizes under physiological conditions to DNA comprising a particular gene or to an mRNA transcript of that gene and thereby, inhibits the transcription of that gene and/or the translation of that mRNA. Antisense molecules are designed so as to interfere with transcription or translation of a target gene upon hybridization with the target gene. Those skilled in the art will recognize that the exact length of the antisense oligonucleotide and its degree of complementarity with its target will depend upon the specific target selected, including the sequence of the target and the particular bases which comprise that sequence.

It is preferred that the antisense oligonucleotide be constructed and arranged so as to bind selectively with the target under physiological conditions, i.e., to hybridize substantially more to the target sequence than to any other sequence in the target cell under physiological conditions.

In order to be sufficiently selective and potent for inhibition, such antisense oligonucleotides should comprise at least 7 (Wagner et al., Nature Biotechnology 14:840-844, 1996) and more preferably, at least 15 consecutive bases which are complementary to the target. Most preferably, the antisense oligonucleotides comprise a complementary sequence of 20-30 bases.

Although oligonucleotides may be chosen which are antisense to any region of the gene or mRNA transcripts, in preferred embodiments the antisense oligonucleotides correspond to N-terminal or 5' upstream sites such as translation initiation, transcription initiation or promoter sites. In addition, 3'-untranslated regions may be targeted. The 3'- untranslated regions are known to contain *cis* acting sequences which act as binding sites for proteins involved in stabilising mRNA molecules. These *cis* acting sites often form hair-loop structures which function to bind said stabilising proteins. A well known example of this form of stability regulation is shown by histone mRNA's, the abundance of which is controlled, at least partially, post-transcriptionally.

The term "antisense oligonucleotides" is to be construed as materials manufactured either *in vitro* using conventional oligonucleotide synthesising methods which are well known in the art or oligonucleotides synthesised recombinantly using expression vector constructs. Modified oligonucleotide is construed in the following manner.

The term "modified oligonucleotide" as used herein describes an oligonucleotide in which;
i) at least two of its nucleotides are covalently linked via a synthetic internucleoside linkage (i.e., a linkage other than a phosphodiester linkage between the 5' end of one nucleotide and the 3' end of another nucleotide). Alternatively or preferrably said linkage may be the 5' end of one nucleotide linked to the 5' end of another nucleotide or the 3' end of one nucleotide with the 3' end of another nucleotide; and/or
ii) a chemical group not normally associated with nucleic acids has been covalently attached to the oligonucleotide or oligoribonucleotide. Preferred synthetic internucleoside linkages are phosphorothioates, alkylphosphonates, phosphorodithioates, phosphate esters, alkylphosphonothioates, phosphoramidates, carbamates, phosphate triesters, acetamidates, peptides, and carboxymethyl esters.

The term "modified oligonucleotide" also encompasses oligonucleotides with a covalently modified base and/or sugar. For example, modified oligonucleotides include oligonucleotides having backbone sugars which are covalently attached to low molecular weight organic groups other than a hydroxyl group at the 3' position and other than a phosphate group at the 5' position. Thus modified oligonucleotides may include a 2'-0-alkylated ribose group. In addition, modified oligonucleotides may include sugars such as arabinose instead of ribose. Modified oligonucleotides also can include base analogs such as C-5 propyne modified bases (Wagner et al., Nature Biotechnology 14:840-844,1996).

The present invention, thus, contemplates pharmaceutical preparations containing viral based vectors including natural and/or modified antisense molecules that are complementary to and hybridizable with, under physiological conditions, nucleic acids encoding proteins the regulation of results in beneficial therapeutic effects, together with pharmaceutically acceptable carriers (eg polymers, liposomes/cationic lipids).

Viral based vectors including antisense oligonucleotides may be administered as part of a pharmaceutical composition. Such a pharmaceutical composition may include the viral based vectors including antisense oligonucleotides in combination with any standard physiologically and/or pharmaceutically acceptable carriers which are known in the art (eg liposomes). The compositions should be sterile and contain a therapeutically effective amount of the viral based vector including antisense oligonucleotides for administration to a patient. The term "pharmaceutically acceptable" means a non-toxic material that does not interfere with the effectiveness of the biological activity of the active ingredients. The term "physiologically acceptable" refers to a non-toxic material that is compatible with a biological system such as a cell, cell culture, tissue, or organism.

In addition viral based vectors are typically combined with carriers, for example polymers, cationic lipids/liposomes.

The use of cationic lipids (eg liposomes, Felgner (1987) Proc.Natl.Acad.Sci USA, 84:p7413) has become a common method to introduce DNA into cells. The cationic head of the lipid associates with the negatively charged nucleic acid backbone of the DNA to be introduced. The lipid/DNA complex associates with the cell membrane and fuses with the cell to introduce the associated DNA into the cell. Liposome mediated DNA transfer has several advantages over existing methods. For example, cells which are recalcitrant to traditional chemical methods are more easily transfected using liposome mediated transfer.

Alternatively, peptide based transfection agents are available, see WO96/41606.

Liposomes are lipid based vesicles which encapsulate a selected viral based vector which is then introduced into a patient. The liposome is manufactured either from pure phospholipid or a mixture of phospholipid and phosphoglyceride. Typically liposomes can be manufactured with diameters of less than 200nm, this enables them to pass through the pulmonary capillary bed. Furthermore the biochemical nature of liposomes confers permeability across blood vessel membranes to gain access to selected tissues. Liposomes do have a relatively short half-life. So called STEALTH^{R} liposomes have been developed which comprise liposomes coated in polyethylene glycol (PEG). The PEG treated liposomes have a significantly increased half-life when administered to a patient. In addition STEALTH^{R} liposomes show reduced uptake in the reticulo-endothelial system and enhanced accumulation selected tissues. So called immuno-liposomes have also been develop which combine lipid based vesicles with an antibody or antibodies, to increase the specificity of the delivery of the vector to a selected cells/tissue.

The use of liposomes as delivery means is described in US 5580575 and US 5542935.

The term "dextrin" means a glucose polymer which is produced by the hydrolysis of starch and which consists of glucose units linked together by means mainly of α-1,4 linkages. Typically dextrins are produced by the hydrolysis of starch obtained from various natural products such as wheat, rice, maize and tapioca. In addition to α-1,4 linkages there may be a proportion of α-1,6 linkages in a particular dextrin, the amount depending on the starch starting material. Since the rate of biodegradability of α-1,6 linkages is typically less than that for α-1,4 linkages, for many applications it is preferred that the percentage of α-1,6 linkages is less than 10% and preferably less than 5%. Any dextrin is a mixture of polyglucose molecules of different chain lengths. As a result, no single number can adequately characterise the molecular weight of such a polymer. Accordingly various averages are used, the most common being the weight average molecular weight (Mw) and the number average molecular weight (Mn). Mw is particularly sensitive to changes in the high molecular weights content of the polymer whilst Mn is largely influenced by changes in the low molecular weight of the polymer.

It is preferred that the Mw of the dextrin is in the range from 1,000 to 200,000, more preferably from 2,000 to 55,000.

The term "degree of polymerisation" (DP) can also be used in connection with polymer mixtures. For a single polymer molecule, DP means the number of polymer units. For a mixture of molecules of different DP's, weight average DP and number average DP correspond to Mw and Mn. In addition DP can also be used to characterise a polymer by referring to the polymer mixture having a certain percentage of polymers of DP greater than a particular number or less than a particular number.

It is preferred that, in the present invention, the dextrin contains more than 15% of polymers of DP greater than 12 and, more preferably, more than 50% of polymers of DP greater than 12.

Preferably the dextrin is present in the solution in an amount of less than 10%.

Preferably the dextrin is present in the solution in an amount selected from: 1% (w/v);2%(w/v); 3%(w/v); 4%(w/v); 5%(w/v); 6%(w/v); 7%(w/v); 8%(w/v);9%(w/v); 10%(w/v).

More preferably the dextrin is present from 2 to 5% by weight, most preferably about 4% by weight.

The present invention provides a composition suitable for delivery of a viral based vector to an animal subject, the composition comprising an aqueous solution or suspension of the viral based vector and dextrin. Preferably 4% dextrin solution is used as a delivery vehicle because of its long IP residence time in man.

Furthermore the present invention provides the use of a composition of the invention to deliver a viral based vector, to target cells in an animal subject.

### DETAILED DESCRIPTION OF THE INVENTION

An embodiment of the invention will now be described by example only and with reference to the following figures:
Figure 1 is a graph of viral stability over time during storage at 4°C for rAAV/dextrin (◆) solution and rAAV/saline (□)samples;
Figures 2 is a graph of viral stability over time during storage at 37°C for rAAV/dextrin (◆)solution and rAAV/saline (□)samples;
Figure 3 is a graph to show the influence of repeated freeze-thawing on viral stability; and
Figure 4 illustrates the effect of dextrin on transfection of COS cells by AD5.CMV-Lac z based vector, Figure 4a shows the effect of dextrin on cell viability by measuring total protein correlated with multipicity of infection (MOI); Figure 4b shows the effect of dextrin (◆) on β-galactosidase activity correlated with increasing MOI compared to PBS (□).

### Materials and Methods

Two reporter constructs were used to monitor the effect of dextrin on transfection effciency. Green Fluroescent Protein (GFP) reporter gene was used in an adeno-associated virus (AAV) vector located in an icodextrin solution. Alternatively, LacZ reporter was used to monitor transfection efficiency.

Transgene expression in normal cells in the peritoneal wall was demonstrated at vector concentrations of from 1 x 10⁸ to 1 x 10¹⁰ PN/ml.

### EXAMPLE 1

### (i) Transfection of tissue culture cells with rAAV encoding a Green Fluorescent Protein (GFP) Reporter Gene.

80% confluent BHK cells in 10cm tissue culture dishes were transfected with a total of 30 µg plasmid DNA per plate using Lipofectin/Peptide 6/DNA complexes. The ratio of rAAV vector plasmid (encoding GFP) to packaging plasmid (encoding necessary replication and packaging signals) was 1:3.

### (ii) Infection with Helpervirus

5 hours post transfection cells were infected at a multiplicity of infection (MOI) of 3 with a herpes helpervirus in complete medium.

### (iii) Harvesting

Approximately 42 hrs after infection cells were harvested by scraping, pelleted by spinning at 3500rpm for 10 min and resuspended in 10ml of buffer (140mM NaCl, 5mM KCl, 0.7mM K₂HPO₄, 25mM TrisHCl-pH 7.4). The solution was freeze thawed four times between a dry ice/ethanol bath and a 37°C waterbath to lyze the cells. The lysate was then clarified from cellular debris by centrifugation at 3500rpm for 10 min.

### (iv) CsCl Density Gradient Purification of rAAV

1) The cleared lysate was adjusted to 1.4g/ml by addition of caesium chloride and distributed into a Beckman Ultra-Clear centrifuge tube.
2) The product was then spun in a Beckman Ultracentrifuge, SW41Ti rotor, at 40000rpm and 20°C for 20-24hrs (brake "OFF" position).
3) The middle region of the tube was collected by side puncture.
4) The density was readjusted and the product transferred, then centrifuged as above.
5) 3 fractions (~2ml each) were collected across the gradient by side puncture with a needle and letting the solution drip into a sterile container.

### v) Dialysis of fractions against icodextrin or saline

Each fraction was divided in two equal portions and dialysed at 4°C against five changes of dextrin or saline respectively (2 litres each change) using dialysis cassettes (Slide A-Lyzer Dialysis Cassettes, 10000 MW cut-off).

### vi) Assay fractions for rAAV

Subconfluent HeLa cells in 96well dishes were infected with 5µl of each fraction diluted in complete media and wildtype Adenovirus (wt Ad) was added to facilitate the infection. After 24 hours cells were screened for GFP expression using an inverted fluorescence microscope. The fraction containing the most rAAV was determined and used for the following experiments.

The fraction containing the most rAAV (in dextrin and saline) was separated into small aliquots. These aliquots were stored at -80°C.

### Example 1 results

i) Storage at 4°C/37°C
   a) 25 µl samples (n=1) were thawed out each day and stored at 4°C and 37°C respectively. After 7 days samples were titred together with an aliquot not exposed to these temperatures (day 0 sample).
   b) The 37°C experiment was repeated and samples (n=3) for both icodextrin and saline were stored for 96 hours and 40 hours. They were titred together with aliquots not exposed to this temperature.
ii) Repeated freeze-thawing
   One aliquot of rAAV/dextrin and rAAV/saline was freeze-thawed repeatedly between dry-ice and 37°C waterbath and 25 µl samples (n=3) were taken after 0, 10 and 20 freeze-thawing cycles. Samples were then titred.

### Titration

1) HeLa cells were seeded in 96well dishes (2 x 10⁴ cells/well) prior to titration experiments to ensure cells were subconfluent.
2) Using 10 µl of each aliquot, tenfold serial dilutions were prepared in complete media in a total volume of 1ml;
- 10 µl of aliquot plus 990 µl of medium gave a 1:100 dilution,
- 100 µl of this 10⁻² dilution was transferred to a second tube containing 900 µl of media, giving a 10⁻³ dilution,
- 100 µl of this 10⁻³ was transferred to a third tube, etc.

3) 50 µl of each dilution was transferred to a second set of 1.5ml tubes and 2 µl of wt Ad (stock 5x10⁹pfu/ml) added before mixing.
4) Media was taken from the cells and rAAV/wtAd mixture was added to the cells.
5) Green cells were counted after 24 hours using an inverted fluorescence microscope.
6) The titre was calculated as follows:

| | |
|---|---|
| 30 green cells/50 µl | in 10⁻⁶ dilution |
| 600 green cells/1000 µl | in 10⁻⁶ dilution |

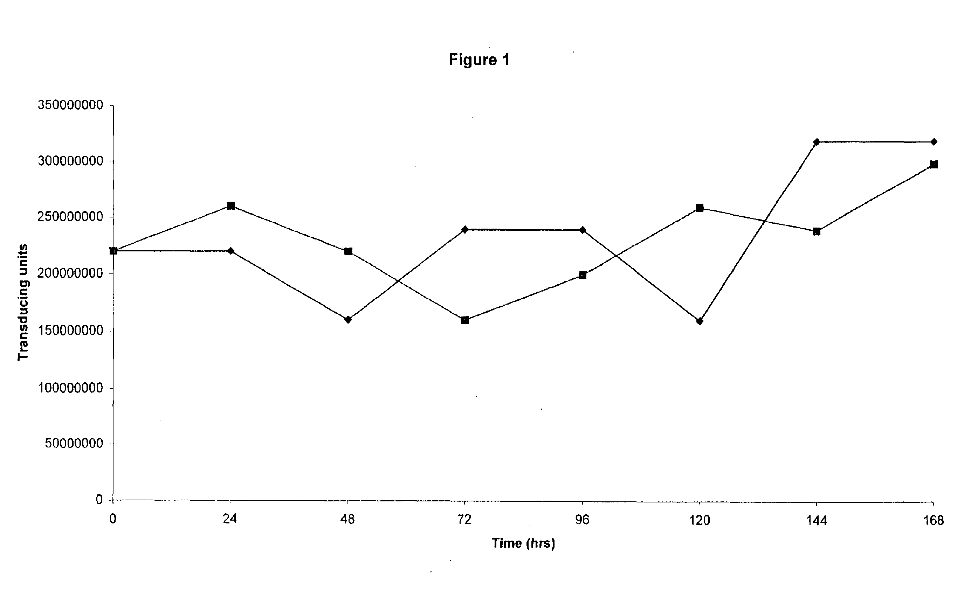

Titre: 600x 10⁶/ml = 6x 10⁸/ml
(If different titres are listed they come from different dilutions) See Figures 1, 2a, 2b and 3.

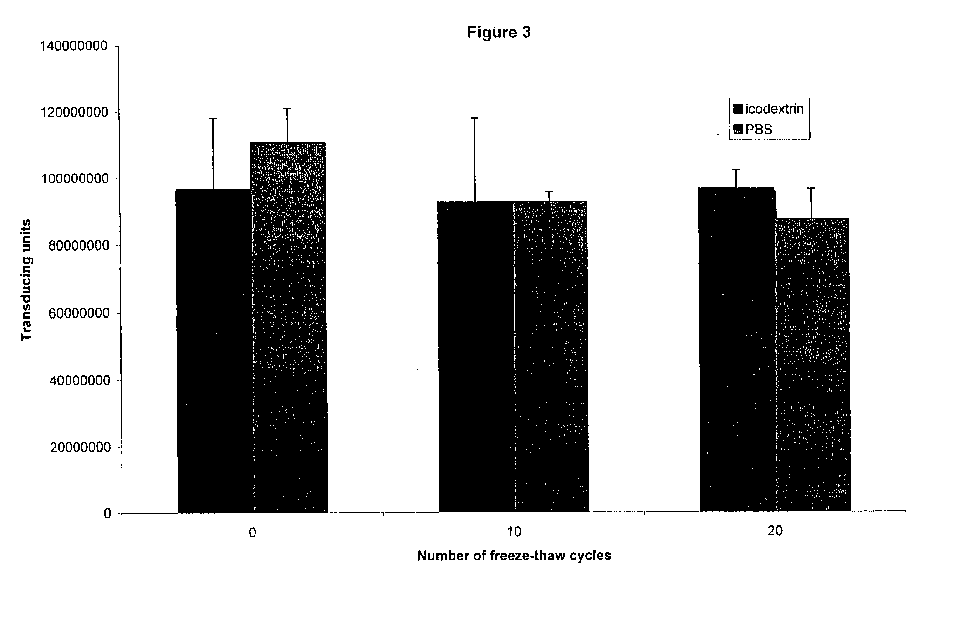

It was possible to freeze thaw the solution up to 20 times with no effect on the stability of the virus (see Figure 3).

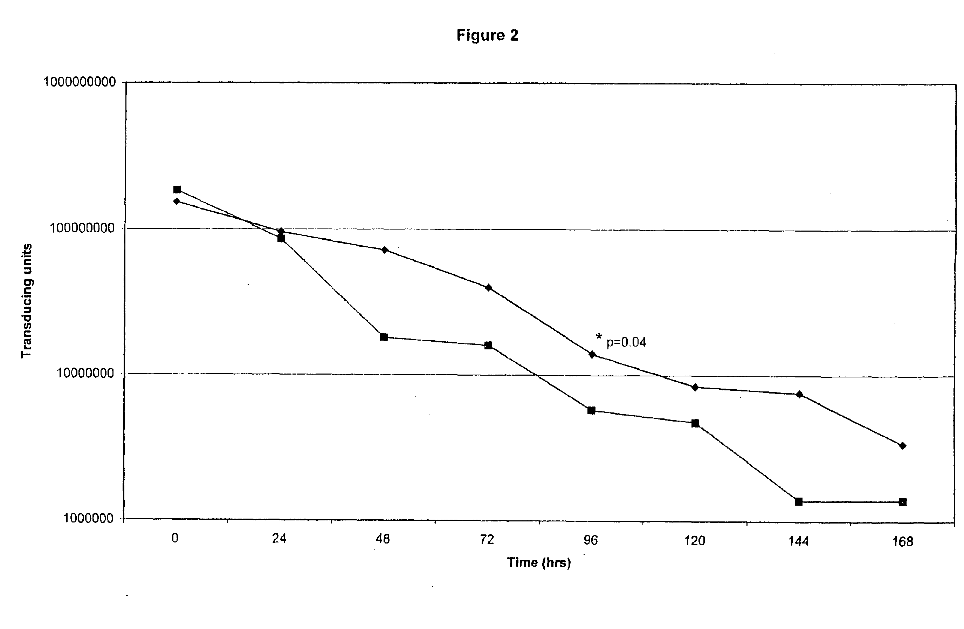

At 4°C there is no difference in virus stability. However, at 37° there is a difference in virus stability between dextrin and saline (Figure 2a). This is clearly demonstrated from the 96 hours data (Figure 2b). This temperature and time range are highly relevant for transfection *in vivo.* This difference was shown to be statistically significant (p = 0.04).

### Example 2

Adenovirus vector Ad5.CMV-LacZ was obtained from Quantum Biotechnologies Inc. USA. Viral titration is monitored by three different methods:
i) OD₂₆₀ : 1 x 10¹¹ viral particles/ml
ii) TCID₅₀ 1.62 x 10¹⁰ TCID/ml (TCID₅₀ = 50% tissue culture infectious dose); and
iii) Plaque assay: 3.25 x 10⁹ PFU/ml (plaque forming units)

COS cells were cultured to a cell density of 5 x 10⁵ cells / well in a 96 well microtitre plate. Cells were cultured under standard cell culture conditions but used heat denatured fetal calf serum. Viral stocks were prepared in dextrin or PBS at a final MOI of 0, 0.1, 0.5, 1, 5, or 10 in dextrin. Final concentration of dextrin is 4%.

### Example 2 results

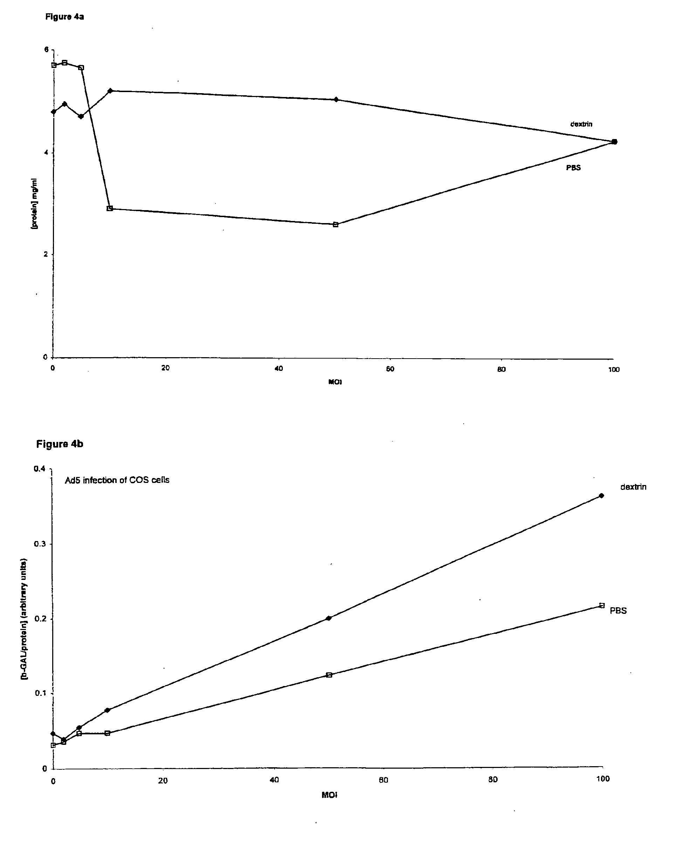

Referring to Figure 4a. 24 hours after addition of the viral vector the cytopathic effects of the virus are more marked in the absence of dextrin. Figure 4b shows that in the presence of 4% dextrin there is an increase in the amount of β-galactosidase produced compared to PBS control.

## Claims

1. A composition comprising a solution of dextrin and a viral based vector comprising therapeutic genetic material **characterised in that** the dextrin has a weight average molecular weight of from 1,000-200,000 for use as a therapeutic composition.

2. A composition according to Claim 1 wherein the dextrin has a weight average molecular weight of from 2000-55,000.

3. A composition according to Claim 1 or 2 wherein said therapeutic genetic material is genomic DNA.

4. A composition according to Claim 1 or 2 wherein said therapeutic genetic material is cDNA.

5. A composition according to any of Claims 1-4 wherein said viral based vector is selected from the following group: adenovirus; adeno-associated virus; herpesvirus; lentivirus, or baculovirus.

6. A composition according to Claim 1 or 2 wherein said therapeutic genetic material is an antisense nucleic acid molecule.

7. A composition according to any of Claims 1-6 wherein said viral based vector is combined with at least one carrier and/or excipient.

8. A composition according to Claim 7 wherein said carrier or excipient is liposome based.

9. A composition according to any of Claims 1-8 wherein said dextrin comprises glucose molecules linked together by equal to or less than 10% α 1-6 linkages.

10. A composition according to any of Claims 1-8 wherein said dextrin comprises glucose molecules linked together by equal to or less than 5% α 1-6 linkages.

11. A composition according to any of Claims 1-10 wherein said dextrin solution consists of at least 15% of polymers with a degree of polymerisation equal to or greater than 12.

12. A composition according to any of Claims 1-10 wherein said dextrin solution consists of at least 50% of polymers with a degree of polymerisation equal to or greater than 12.

13. A composition according to any of Claims 1-12 wherein said dextrin solution is less than 10% (w/v) dextrin.

14. A composition according to any of Claims 1-12 wherein said dextrin solution is 10% (w/v) dextrin.

15. A composition according to any of Claims 1-12 wherein said dextrin solution is at least 5% (w/v) dextrin.

16. A composition according to any of Claims 1-12 **characterised in that** said dextrin solution is 4% (w/v) dextrin.

17. A product comprising dextrin that has a weight average molecular weight of from 1000-200,000 and therapeutic genetic material wherein the material comprises a viral based vector.

## Patentansprüche

1. Zubereitung, umfassend eine Lösung von Dextrin und einem Virus-basierten Vektor, umfassend therapeutisches genetisches Material, **dadurch gekennzeichnet, dass** das Dextrin ein Gewichtsmittel des Molekulargewichts von 1.000 bis 200.000 aufweist, zur Verwendung als therapeutische Zubereitung.

2. Zubereitung nach Anspruch 1, worin das Dextrin ein Gewichtsmittel des Molekulargewichts von 2.000 bis 55.000 aufweist.

3. Zubereitung nach Anspruch 1 oder 2, worin das therapeutische genetische Material genomische DNS ist.

4. Zubereitung nach Anspruch 1 oder 2, worin das therapeutische genetische Material cDNS ist.

5. Zubereitung nach einem der Ansprüche 1 bis 4, worin der Virus-basierte Vektor gewählt ist aus der folgenden Gruppe: Adeno-Virus, Adeno-assoziierter Virus, HerpesVirus, Lentivirus oder Baculovirus.

6. Zubereitung nach Anspruch 1 oder 2, worin das therapeutische genetische Material ein Antisense-Nucleinsäure-Molekül ist.

7. Zubereitung nach einem der Ansprüche 1 bis 6, worin der Virus-basierte Vektor mit wenigstens einem Träger und/oder Hilfsstoff kombiniert ist.

8. Zubereitung nach Anspruch 7, worin der Träger oder Hilfsstoff Liposomen-basiert ist.

9. Zubereitung nach einem der Ansprüche 1 bis 8, worin das Dextrin Glucosemoleküle umfasst, die miteinander verbunden sind über α-1,6-Bindungen in einer Menge von gleich oder weniger als 10 %.

10. Zubereitung nach einem der Ansprüche 1 bis 8, worin das Dextrin Glucosemoleküle umfasst, die miteinander verbunden sind über α-1,6-Bindungen in einer Menge von gleich oder weniger als 5 %.

11. Zubereitung nach einem der Ansprüche 1 bis 10, worin die Dextrin-Lösung besteht aus wenigstens 15 % Polymeren mit einem Polymerisations-Grad gleich oder größer als 12.

12. Zubereitung nach einem der Ansprüche 1 bis 10, worin die Dextrin-Lösung besteht aus wenigstens 50 % Polymeren mit einem Polymerisations-Grad gleich oder größer als 12.

13. Zubereitung nach einem der Ansprüche 1 bis 12, worin die Dextrin-Lösung weniger als 10 % (Gewicht/Volumen) Dextrin aufweist.

14. Zubereitung nach einem der Ansprüche 1 bis 12, worin die Dextrin-Lösung 10 % (Gewicht/Volumen) Dextrin aufweist.

15. Zubereitung nach einem der Ansprüche 1 bis 12, worin die Dextrin-Lösung wenigstens 5 % (Gewicht/Volumen) Dextrin aufweist.

16. Zubereitung nach einem der Ansprüche 1 bis 12, **dadurch gekennzeichnet, dass** die Dextrin-Lösung 4 % (Gewicht/Volumen) Dextrin aufweist.

17. Produkt, umfassend Dextrin, das ein Gewichtsmittel des Molekulargewichts von 1.000 bis 200.000 aufweist, und ein therapeutisches genetisches Material, worin das Material einen Virus-basierten Vektor umfasst.

## Revendications

1. Composition comprenant une solution de dextrine et d'un vecteur à base virale comprenant un matériel génétique thérapeutique, **caractérisée en ce que** la dextrine a une moyenne en poids du poids moléculaire de 1000 à 200 000, à des fins d'utilisation comme composition thérapeutique.

2. Composition suivant la revendication 1, dans laquelle la dextrine a une moyenne en poids du poids moléculaire de 2000 à 55 000.

3. Composition suivant la revendication 1 ou 2, dans laquelle ledit matériel génétique thérapeutique est un ADN génomique.

4. Composition suivant la revendication 1 ou 2, dans laquelle ledit matériel génétique thérapeutique est un ADNc.

5. Composition suivant l'une quelconque des revendications 1 à 4, dans laquelle ledit vecteur à base virale est choisi dans le groupe suivant : un adénovirus ; un virus adéno-associé ; un herpèsvirus ; un lentivirus et un baculovirus.

6. Composition suivant la revendication 1 ou 2, dans laquelle ledit matériel génétique thérapeutique est une molécule d'acide nucléique antisens.

7. Composition suivant l'une quelconque des revendications 1 à 6, dans laquelle ledit vecteur à base virale est combiné avec au moins un support et/ou excipient.

8. Composition suivant la revendication 7, dans laquelle ledit support ou excipient est à base de liposomes.

9. Composition suivant l'une quelconque des revendications 1 à 8, dans laquelle ladite dextrine comprend des molécules de glucose liées ensemble par une proportion égale ou inférieure à 10 % de liaisons α-1,6.

10. Composition suivant l'une quelconque des revendications 1 à 8, dans laquelle ladite dextrine comprend des molécules de glucose liées ensemble par une proportion égale ou inférieure à 5 % de liaisons α-1,6.

11. Composition suivant l'une quelconque des revendications 1 à 10, dans laquelle ladite solution de dextrine consiste en au moins 15 % de polymères ayant un degré de polymérisation égal ou supérieur à 12.

12. Composition suivant l'une quelconque des revendications 1 à 10, dans laquelle ladite solution de dextrine consiste en au moins 50 % de polymères ayant un degré de polymérisation égal ou supérieur à 12.

13. Composition suivant l'une quelconque des revendications 1 à 12, dans laquelle ladite solution de dextrine comprend moins de 10 % (en poids/volume) de dextrine.

14. Composition suivant l'une quelconque des revendications 1 à 12, dans laquelle ladite solution de dextrine comprend 10 % (en poids/volume) de dextrine.

15. Composition suivant l'une quelconque des revendications 1 à 12, dans laquelle ladite solution de dextrine comprend au moins 5 % (en poids/volume) de dextrine.

16. Composition suivant l'une quelconque des revendications 1 à 12, **caractérisée en ce que** ladite solution de dextrine comprend 4 % (en poids/volume) de dextrine.

17. Produit comprenant de la dextrine qui a une moyenne en poids du poids moléculaire 1000 à 200 000 et un matériel génétique thérapeutique, dans lequel le matériel comprend un vecteur à base virale.
